# EUROPEAN PATENT APPLICATION

(11) **EP 4 495 243 A1**
(43) Date of publication of application: **22.01.2025**
(21) Application number: 23770705.4
(22) Date of filing: 13.03.2023
(51) Int. Cl.: C12N 15/12, A61K 9/50, A61K 47/44, A61K 48/00, C12N 5/10, C12N 15/11, C12N 15/85

(54) **NOVEL METHOD FOR PRODUCING EXOSOME CONTAINING TARGET NUCLEIC ACID AND SERVING AS NUCLEIC ACID MEDICINE**

(30) Priority: 15.03.2022 JP 2022040244
(71) Applicant: ProgenicyteJapan Co., Ltd., Kobe-city, Hyogo 650-0046 (JP)
(72) Inventor: SUH, Neung, Kobe-city, Hyogo 6500046 (JP)
(74) Representative: Elion IP, S.L.
(86) International application number: PCT/JP2023/009529
(87) International publication number: WO 2023/176750

(57) **Abstract**

The purpose of the present disclosure is to provide a novel DDS technology for delivering a nucleic acid to a target cell. The present disclosure relates to a method for producing an exosome containing a target nucleic acid, the method being characterized by including: a step for preparing a recombinant host cell by introducing a polynucleotide containing the nucleic acid sequence of a target nucleic acid and a signal nucleic acid sequence represented by SEQ ID NO: 1 into a host cell; and a step for culturing the aforementioned recombinant host cell and causing the host cell to produce and secrete an exosome containing the target nucleic acid. In addition, the present disclosure also relates to: a polynucleotide containing the nucleic acid sequence of a target nucleic acid and a signal nucleic acid sequence represented by SEQ ID NO: 1; a vector having such a polynucleotide; and an exosome containing the polynucleotide containing the nucleic acid sequence of the target nucleic acid and the signal nucleic acid sequence represented by SEQ ID NO: 1.

## Description

### TECHNICAL FIELD

The present invention relates to a novel method for producing exosomes as nucleic acid medicines loaded with a target nucleic acid.

### BACKGROUND

Nucleic acid medicines have long been attracting attention as the next generation of polymer medicines following proteins, but nucleic acids are easily degraded in the body in their original form, nucleic acids themselves induce strong immune reactions in the body, and target cell Problems include the difficulty of efficiently delivering the information internally. DDS (Drug Delivery System) is a means to solve these problems, and it is essential for the spread of nucleic acid drugs to prevent nucleic acid degradation, protect against immune reactions, and promote uptake into target tissue cells. Conceivable.

Nucleic acids have very high potential for medical applications, and in particular, gene regulation, which has been difficult to target with conventional low-molecular compounds and proteins, can be achieved with high specificity by directly targeting gene sequences. A therapeutic effect is expected. Nucleic acid medicines are currently being developed around the world, and while the number of seeds available is vast, the reality is that, as mentioned above, the development of DDS that delivers nucleic acids to target cells is lagging behind.

On the other hand, exosomes are granules that are naturally secreted from various cells and are covered with a lipid membrane that is the same as the cell membrane, and contain substances derived from the cytoplasm. The internal substances covered by the lipid membrane are not only protected from metabolic enzymes, but also can freely pass through tissues and cell membranes. There are various secreted cell-derived membrane proteins on the surface of the exosome membrane, and by being taken up by other specific cells through these membrane proteins and releasing internal substances, It is believed to be responsible for transmitting information. Through this series of processes, exosomes are able to transport substances to distant cells, making them ideal for intracellular DDS of pharmaceuticals. Currently, basic research on using exosomes as nucleic acid carriers is continuing all over the world, but the only reported cases are that nucleic acids are artificially added to recovered exosomes using existing methods (electroporation, lipofection, etc.). The main method is to introduce exosomes manually, and there are many problems from the viewpoint of introduction efficiency and quality of exosomes (see Patent Documents 1 and 2 and Non-Patent Documents 1 and 2). Another disadvantage is that it is difficult to mass produce and control quality as a pharmaceutical product.

### Prior art document

### Patent Literature

Patent Document 1: JP 2019-122309
Patent Document 2: JP 2020-023503

### Non-Patent Literature

Non Patent Literature 1: Mol. Pharm, 2015, 12, 10, 3650-3657
Non-Patent Document 2: Cancer Letters, 2021, 505, 58-72

### SUMMARY

### Problems to be solved by the invention

The purpose of the present invention is to provide a novel DDS technique for delivering a desired nucleic acid to a target tissue and a target cell. Specifically, the purpose of the present invention is to provide a novel method for producing exosomes on which a desired nucleic acid is mounted, a vector and a vector system used in the method, and a pharmaceutical composition comprising exosomes produced by the method.

### Means for solving the Problem

It was discovered that by analyzing exosomes present in blood or body fluid, the exosome is not only the protein or RNA of the cytoplasm reported to the exosome but also the DNA, and the exosomes of the cancer patient include a large amount of DNA. When the nucleic acid sequence of DNA contained in exosomes secreted by cancer stem cells was analyzed, it was found that a new signal nucleic acid sequence having no report was inserted into the non-coding region. On the other hand, when a cytoplasm DNA is examined, they have been detected that do not have this special nucleic acid sequence. However, DNA contained in exosomes was considered to be selectively taken into exosomes by having this nucleic acid sequence at most having this special nucleic acid sequence. This DNA was not a waste from the genome, but it was strongly suggested that the gene is transferred to exosomes by being inserted when RNA is reversely transcribed with cytoplasm. Therefore, the inventors have developed a technique capable of efficiently capturing specific nucleic acids into exosomes in human cells (mesenchymal stem cells, human fetal kidney cells, etc.) using this new knowledge. As a result, by using this technique, exosomes containing a desired nucleic acid were successfully prepared. According to the present invention, a novel DDS technique for delivering a desired nucleic acid to a target tissue and a target cell can be provided. That is, the gist of the present invention is as follows.
[1] A method for producing exosomes containing a target nucleic acid,
   A nucleic acid sequence of a target nucleic acid and a polynucleotide comprising a signal nucleic acid sequence represented by SEQ ID NO: 1 are introduced into a host cell to prepare a recombinant host cell; and
   The recombinant host cells are cultured to produce and secrete exosomes containing the target nucleic acid
   The method for producing exosomes is characterized by having the exosomes.
[2] A polynucleotide comprising a nucleic acid sequence of a target nucleic acid and a signal nucleic acid sequence represented by SEQ ID NO: 1.
[3] A vector comprising a nucleic acid sequence of a target nucleic acid and a polynucleotide including a signal nucleic acid sequence represented by SEQ ID NO: 1.
[4] An expression vector system capable of cloning in a form in which a signal nucleic acid sequence represented by SEQ ID NO: 1 is inserted into a target nucleic acid.
[5] A recombinant host cell into which a polynucleotide containing a nucleic acid sequence of a target nucleic acid and a signal nucleic acid sequence represented by SEQ ID NO: 1 is introduced.
[6] The recombinant host cell described in [5], which is for producing exosomes containing a target nucleic acid.
[7] Exosome having a polynucleotide comprising a nucleic acid sequence of a target nucleic acid and a signal nucleic acid sequence represented by SEQ ID NO: 1.
[8] Exosome produced by the production method described in [1]
[9] A pharmaceutical composition comprising exosomes described in [9] [7] or [8].

### Effect of the Invention

According to the present invention, a target nucleic acid (target nucleic acid) can be cloned in an upstream region of a non-coding region into which a signal nucleic acid sequence (ELS) represented by SEQ ID NO: 1 is inserted, and the target nucleic acid can be introduced into an exosome produced by the cell by transfecting the nucleic acid to the host cell. According to the present invention, a specific nucleic acid can be efficiently taken into exosomes in a host cell, and exosomes on which a desired nucleic acid is mounted can be freely prepared. Exosomes obtained by the present invention may be suitably used as a new DDS technique for delivering a desired nucleic acid to a target tissue and a target cell.

The exosomes are granules covered with a lipid membrane of the same quality as a cell membrane naturally secreted from various cells, and are excellent in safety and stability in the body because a membrane protein that realizes immune tolerance is expressed. Further, in the case of a pharmaceutical that acts on cells in the brain, the administered DDS body needs to exceed the Brain Blood Barrier (BBB), but the exosomes are covered with the same lipid membrane as the cell membrane, so that BBB can be efficiently passed, and there is no restriction on the target organ of the existing DDS. Further, by expressing a membrane protein that acts on interaction with cells expressed in the membrane, exosomes that have taken the desired nucleic acid of the present invention are thought to be dominant even in that it is possible to control uptake into a specific tissue and a cell.

### BRIEF DESCRIPTION OF THE DRAWINGS

FIG. 1 is a diagram illustrating a construct of a GFP expression cassette (pPJ4) having 3'UTR containing ELS downstream of a GFP/Neo fusion protein gene and a GFP expression cassette (pPJ3) having 3'UTR that does not include ELS as a control.
FIG. 2-1 shows a construct (pPJ5) that does not include ELS constructed by inserting a GFP expression cassette having a 3'UTR including ELS downstream of a GFP/Neo fusion protein gene, and a 3'UTR-free GFP expression cassette not including ELS as control, on the downstream of the UbC promoter on the lentivirus vector in a direction opposite to the transfer direction.
FIG. 2-2 is a diagram illustrating a construct (pPJ6) including ELS constructed by inserting a GFP expression cassette having a 3'UTR including ELS downstream of a GFP/Neo fusion protein gene and a 3'UTR-containing GFP expression cassette not including ELS as control, downstream of the UbC promoter on the lentivirus vector in a direction opposite to the transfer direction.
FIG. 3 is a diagram illustrating a confirmation result of a 3'UTR region of NANOC7 on Clone 7 A and Clone 7 B by PCR.
FIG. 4 is a diagram illustrating a confirmation result of the construct of the obtained clone (pPJ1) after cloning the CMV promoter and the EGFP/Neo fusion gene into the pUC19.
FIG. 5 is a diagram illustrating a confirmation result of the construct of the clone (pPJ3 (ELS-free) and pPJ4 (ELS-containing)) obtained after cloning the 3'UTR of NANOGP8 (ELS-free and ELS-containing) into pPJ1.
FIG. 6 is a diagram illustrating a result of confirming a construct of the obtained clone (pPJ2) after removing the EGFP gene in the lentiviral vector pUltra # 241291.
FIG. 7 is a diagram illustrating a confirmation result of the construct of the clone (pPJ5 (ELS-free) and pPJ6 (ELS-containing)) obtained after cloning the EGFP expression cassettes derived from pPJ3 (ELS-free) and pPJ4 (ELS) into pPJ2, respectively.
In FIG. 8, (a) is a schematic diagram of the constructed plasmid vectors (pPJ3, pPJ4). (b) is a fluorescence microscopic photograph of HEK 293 cells at 48 hours after the introduction of each plasmid vector by lipofection. These are the results of observation using an all-in-one fluorescence microscope (Keyence Corporation) (×4).
FIG. 9 is a schematic diagram of pPJ3 (plasmid 3) and PJ4 (plasmid 4).
FIG. 10 is a schematic diagram of pLP1, pLP2, and pLP/VSVG.
In FIG. 11, (a) is a schematic diagram of a constructed lentiviral vector (pPJ5, pPJ6). (b) is a fluorescence microscope photograph of HEK 293 cells of 48 hours after introduction of each vector by lipofection. These are the results of observation using an all-in-one fluorescence microscope (Keyence Corporation) (×10).
FIG. 12-1 is a schematic diagram of a recipe envelope vector used for CMVp-VN 173-NEO fusion.
FIG. 12-2 is a diagram illustrating a cloning site for the MCS of pUC 18 of CMVp-VN 173-NEO fragment into MCS of pUC18. (Hincll-Xbal restriction enzyme site)
FIG. 12-3 is a diagram illustrating a flow of construction of plasmid pPJ4 including 3'UTR (ELS-free) derived from Clone 7A and 3'UTR (ELS-containing) derived from Clone 7B.
FIG. 12-4 is a diagram illustrating a flow of cloning of a GFP expression cassette to a lentivirus vector.
FIG. 13 is a diagram schematically illustrating a step of cloning a target nucleic acid (target nucleic acid) in an upstream region of a non-coding region into which the ELS is inserted, and transfecting the target nucleic acid to the host cell by the method according to the present invention.
FIG. 14 is a schematic diagram of an expression cassette including 3'UTR including a CMV promoter, an EGFP gene, and a 3'UTR containing a 22mer Exosome Localization Signal (ELS) (SEQ ID NO: 1).
FIG. 15 is a diagram showing the results of PCR confirmation of the EGFP gene incorporated into exosomes.
FIG. 16 is a fluorescence micrograph of HEK293 cells in which EGFP was expressed intracellularly by treatment with exosomes containing EGFP-DNA.

### EMBODIMENTS FOR CARRYING OUT THE INVENTION

The present invention will be explained in detail below. In this specification, unless otherwise specified, molecular biological techniques can be carried out by methods described in general laboratory books known to those skilled in the art or methods analogous thereto. In addition, the terms used herein are interpreted as commonly used in the art unless otherwise specified.

The present invention provides a step of preparing a recombinant host cell by introducing a polynucleotide comprising a nucleic acid sequence of a target nucleic acid and a signal nucleic acid sequence represented by SEQ ID NO: 1 into a host cell, and a step of preparing a recombinant host cell. The present invention relates to a method for producing exosomes containing a target nucleic acid, which comprises the steps of culturing, producing and secreting exosomes containing the target nucleic acid.

The present invention also relates to a polynucleotide comprising a nucleic acid sequence of a target nucleic acid and a signal nucleic acid sequence represented by SEQ ID NO: 1.

The present invention also relates to a vector having a polynucleotide comprising a nucleic acid sequence of a target nucleic acid and a signal nucleic acid sequence represented by SEQ ID NO: 1.

The present invention also relates to an expression vector system that allows cloning of a target nucleic acid with a signal nucleic acid sequence represented by SEQ ID NO: 1 inserted therein.

The present invention also relates to a recombinant host cell into which a polynucleotide containing a nucleic acid sequence of a target nucleic acid and a signal nucleic acid sequence represented by SEQ ID NO: 1 has been introduced, and this recombinant host cell has a nucleic acid sequence of a target nucleic acid. It is suitably used for producing exosomes containing.

The present invention also relates to an exosome having a polynucleotide comprising a nucleic acid sequence of a target nucleic acid and a signal nucleic acid sequence represented by SEQ ID NO: 1.

The present invention provides a step of preparing a recombinant host cell by introducing a polynucleotide comprising a nucleic acid sequence of a target nucleic acid and a signal nucleic acid sequence represented by SEQ ID NO: 1 into a host cell, and a step of preparing a recombinant host cell. The present invention also relates to exosomes produced by a method for producing exosomes containing a target nucleic acid, which comprises the steps of culturing, producing and secreting exosomes containing the target nucleic acid.

The present invention provides a step of preparing a recombinant host cell by introducing a polynucleotide comprising a nucleic acid sequence of a target nucleic acid and a signal nucleic acid sequence represented by SEQ ID NO: 1 into a host cell, and a step of preparing a recombinant host cell. It also relates to a pharmaceutical composition containing exosomes produced by a method for producing exosomes containing a target nucleic acid, which comprises the steps of culturing, producing and secreting exosomes containing the target nucleic acid.

The present invention is a method for loading exosomes with a target nucleic acid, in which a polynucleotide containing the nucleic acid sequence of the target nucleic acid and a signal nucleic acid sequence represented by SEQ ID NO: 1 is introduced into a host cell, and then assembled into a host cell. The present invention also relates to a method for loading exosomes with a target nucleic acid, which comprises the steps of preparing a recombinant host cell, and culturing the recombinant host cell to produce and secrete an exosome containing the target nucleic acid.

The present invention further relates to a method for producing exosomes that combines the above-described method of loading a target nucleic acid onto an exosome and a method of expressing a specific membrane protein that serves as a targeting moiety on the surface of the exosome.

### (Definitions)

All scientific and technical terms used in this application, as stated above, are to be interpreted as commonly used in the art, unless otherwise stated. It should be noted that, when used in this application, the following terms have the meanings specified.

As used herein, the term "polynucleotide" is used interchangeably with "nucleic acid," "gene," or "nucleic acid molecule," and refers to a polymer of nucleotides, in either single-stranded or double-stranded form. refers to a series of nucleotides, deoxyribonucleotides or ribonucleotides, and includes, unless otherwise specified, known analogs of natural nucleotides that hybridize to nucleic acids in a manner similar to naturally occurring nucleotides. As used herein, the term "nucleic acid sequence" is used interchangeably with "nucleotide sequence" or "base sequence" and is denoted as a sequence of deoxyribonucleotides (abbreviated as A, G, C and T). For example, in the present invention, "a polynucleotide comprising a signal nucleic acid sequence represented by SEQ ID NO: 1" refers to a polynucleotide comprising a sequence represented by each deoxynucleotide A, G, C and/or T of SEQ ID NO: 1. means.

The term "vector" refers to a construct capable of delivering, preferably expressing, one or more genes or sequences of interest within a host cell.

The term "foreign gene" refers to a structural gene introduced by a transformation operation, etc., rather than a structural gene originally possessed by the host (a structural gene contained in the chromosome of the natural host before transformation). means. Even if the host naturally possesses a gene, a gene introduced by a transformation operation or the like is a foreign gene in the present invention.

"Exosomes" are granules covered with a lipid membrane that is the same as the cell membrane and are naturally secreted from various cells. Membrane vesicles derived from cytosis. It is also present in large amounts in all body fluids such as blood, saliva, and urine. Exosomes also typically have a diameter of 30 nm to 150 nm, but may contain membrane vesicles up to 200 nm in diameter. Also called exosome.

Exosomes contain lipids and proteins derived from cell membranes on their surface, and the inside contains nucleic acids and proteins such as mRNA, miRNA, and DNA, and contains information derived from the released cell. It is known that these biological information molecules consisting of nucleic acids and proteins function in cells that have taken up exosomes, and it has been revealed that intercellular communication occurs through the exchange of nucleic acids and proteins via exosomes. Therefore, it is thought that exosomes can be used for disease prevention and treatment.

The present invention will be specifically explained below.

### (Method for producing exosomes containing a target nucleic acid)

One embodiment of the present invention is a method for producing exosomes containing a target nucleic acid. Specifically, the method for producing exosomes of the present invention involves introducing a polynucleotide containing the nucleic acid sequence of the target nucleic acid and a signal nucleic acid sequence represented by SEQ ID NO: 1 into a host cell, and culturing the recombinant host cell. (hereinafter simply referred to as "recombinant host cell preparation step"), and a step of culturing the recombinant host cells to produce and secrete exosomes containing the target nucleic acid (hereinafter simply referred to as "exosome preparation step"). production/secretion process). According to the method for producing exosomes of the present invention, a target nucleic acid can be efficiently incorporated into exosomes within a host cell, and exosomes incorporating a desired nucleic acid can be freely prepared. Exosomes obtained by the method of the present invention can be suitably used as a new DDS technology to deliver desired nucleic acids to target cells. Here, in the present invention, the signal nucleic acid sequence refers to the sequence represented by SEQ ID NO: 1, and since the presence of this sequence allows the target nucleic acid to be efficiently incorporated into exosomes, the Exosome Localization Signal Also called (ELS).

The method for producing exosomes of the present invention will be explained step by step.

### [Process for preparing recombinant host cells]

In this step, a polynucleotide containing the nucleic acid sequence of the target nucleic acid and a signal nucleic acid sequence (ELS) represented by SEQ ID NO: 1 is introduced into a host cell, and a recombinant host cell is prepared. This is a step of preparing cells. When a polynucleotide containing the nucleic acid sequence of the target nucleic acid and the nucleic acid sequence with ELS inserted into the non-coding region is introduced into a host cell, the nucleic acid is selectively taken up into exosomes within the host cell, and the nucleic acid is taken up. Exosomes are produced and secreted into the culture medium. The position where the ELS is inserted is not particularly limited as long as it is within the non-coding region, but it is preferably within the 3'UTR downstream of the nucleic acid of interest.

That is, the polynucleotide used in this step contains the nucleic acid sequence of the target nucleic acid and the signal nucleic acid sequence represented by SEQ ID NO: 1, and the signal nucleic acid sequence represented by SEQ ID NO: 1 is located within the non-coding region. It is preferable to be located at . Furthermore, the polynucleotide includes a nucleic acid sequence of a target nucleic acid and a signal nucleic acid sequence represented by SEQ ID NO: 1, and the signal nucleic acid sequence represented by SEQ ID NO: 1 is located in the downstream 3'UTR of the nucleic acid. More preferably, it is a polynucleotide located within. Note that the non-coding region may be a non-coding region of any gene.

The signal nucleic acid sequence (ELS) represented by SEQ ID NO: 1 is a sequence found as a specific 22-bp sequence that is frequently included in DNA in exosomes secreted by cancer stem cells, and is a sequence that is found to be a specific 22-bp sequence that is frequently included in DNA in exosomes secreted by cancer stem cells. By introducing into a host cell a polynucleotide in which a non-coding region sequence to which this ELS sequence has been added (inserted) is linked, the above nucleic acid is selectively incorporated into exosomes within the introduced host cell. This is the first finding by the present inventors.

Regarding the signal nucleic acid sequence (ELS) represented by SEQ ID NO: 1, one or more (for example, 1 to 7, 1 to 6, 1 to 5, 1 to 4, 1 Even if a polynucleotide has a deletion, substitution, insertion, and/or addition of up to 3 nucleotides, 1 to 2 nucleotides, 1 nucleotide, etc., when it is introduced into a vector and transformed into a host cell, the host cell Any polynucleotide that can selectively incorporate a target nucleic acid into exosomes within cells is included within the scope of the present invention. Furthermore, combinations of two or more of these deletions, substitutions, insertions, and additions may be included at the same time. Generally, the smaller the number of nucleotide deletions, substitutions, insertions, and/or additions, the better. Preferred polynucleotides in the present invention also include polynucleotides capable of hybridizing to its complementary sequence of SEQ ID NO: 1 under stringent hybridization conditions, for example.

Here, "stringent conditions" may be any of low stringency conditions, medium stringency conditions, and high stringency conditions. "Low stringency conditions" are, for example, 5x SSC, 5x Denhardt's solution, 0.5% SDS, 50% formamide, and 32°C. Moreover, "medium stringent conditions" are, for example, 5x SSC, 5x Denhardt's solution, 0.5% SDS, 50% formamide, and 42°C. "Highly stringent conditions" are, for example, 5x SSC, 5x Denhardt's solution, 0.5% SDS, 50% formamide, and 50°C. Under these conditions, it can be expected that DNA with higher homology can be obtained more efficiently as the temperature is raised. However, there are multiple factors that can affect the stringency of hybridization, such as temperature, probe concentration, probe length, ionic strength, time, and salt concentration, and those skilled in the art can appropriately select these factors. It is possible to achieve similar stringency with

The "polynucleotide containing the nucleic acid sequence of the target nucleic acid and the signal nucleic acid sequence (ELS) represented by SEQ ID NO: 1" preferably further includes an operably linked promoter sequence. It may also include known sequences such as enhancer sequences, Kozak sequences, appropriate polyadenylation signal sequences, etc. that assist in translation for , and is used for transcription of mRNA to protein, translation to protein and the like.

Note that the "nucleic acid" in "target nucleic acid" is DNA. Furthermore, the "target nucleic acid" refers to a nucleic acid that is desired to be incorporated into exosomes, and may be a nucleic acid derived from a foreign gene or an artificially designed nucleic acid, and is not particularly limited. Examples of "target nucleic acids" include templates for making mRNA that generally performs protein expression, and DNAs that function to regulate the expression and/or activity of specific genes and proteins and the like (such as antisense DNA or the like) and RNA (antisense RNA, RNA interference (RNAi) molecules such as siRNA, miRNA, and shRNA, ribozymes), and gene cassettes that serve as templates for creating CRISPR/Cas, and the like. In the present invention, rather than using RNA itself as a nucleic acid drug, by incorporating a gene cassette containing a promoter (CMV, U6, H1, etc.) into exosomes as DNA, RNA is generated within the cells to which the exosomes are delivered. This allows large amounts of RNA to be produced in vivo, making it more effective than direct delivery of RNA. Medical applications include DNA vaccines, delivery of normal genes for enzymes that do not function due to mutations in genetic diseases, and delivery of genes to stem cells to control cell differentiation (e.g., Hath 1/Atoh1 to inner ear cells). , Nurr1 induces differentiation into dopaminergic neurons, LXh8 induces differentiation into cholinergic neurons, etc. from stem cells). Furthermore, exosomes incorporating these nucleic acids can be effectively used as nucleic acid medicines for the prevention and/or treatment of various diseases such as cancer and viral infections including COVID-19.

The method of introducing "the polynucleotide containing the nucleic acid sequence of the target nucleic acid and the signal nucleic acid sequence (ELS) represented by SEQ ID NO: 1" into host cells is not particularly limited, and examples include viruses, plasmids, artificial chromosomes, etc. Techniques such as lipofection, liposomes, microinjection, etc. can be used. Among these, it is preferable to use vectors from the viewpoint of excellent introduction efficiency and secretion efficiency of exosomes from host cells after introduction, and the vectors are not particularly limited as long as they can efficiently deliver nucleic acids to host cells.. Such vectors include, for example, lentivirus vectors, adeno-associated virus vectors, adenovirus vectors, herpesvirus vectors (e.g., herpes simplex virus vectors), poxvirus vectors, baculovirus vectors, papillomavirus vectors, papovavirus vectors. Examples include viral vectors such as vectors (eg, SV40); plasmid vectors; phagemid vectors; cosmid vectors; bacteriophages such as lambda phage and M13 phage. Among these, for the purpose of permanently expressing a gene, lentivirus vectors, adeno-associated virus vectors, and adenovirus vectors are preferred, lentivirus vectors and adeno-associated virus vectors are more preferred, and lentivirus vectors are even more preferred.. Furthermore, when the purpose is to transiently express a gene, it is preferable to use a plasmid vector.

The above-mentioned vector can contain control sequences such as a promoter, an enhancer, a ribosome binding sequence, a terminator, and a polyadenylation site so that the gene of interest can be expressed. In addition, if necessary, drug resistance genes (e.g. kanamycin resistance gene, ampicillin resistance gene, puromycin resistance gene, etc.), selection marker sequences such as thymidine kinase gene, diphtheria toxin gene, mCherry (red fluorescent protein), green fluorescent protein, etc. (GFP), β-glucuronidase (GUS), and a reporter gene sequence such as FLAG.

The host cell into which "the polynucleotide containing the nucleic acid sequence of the target nucleic acid and the signal nucleic acid sequence (ELS) represented by SEQ ID NO: 1" is introduced is not particularly limited as long as it is a cell that produces and secretes exosomes. Various cells such as natural cells or artificially established cells commonly used in the technical field of the present invention can be used, such as animal cells, insect cells, plant cells, bacteria, yeast, and the like. The type of host cell is preferably selected depending on the intended use of the obtained exosome, and in the case of medical use, it is selected depending on the target species. For example, when the obtained exosomes are used for medicine aimed at humans, the host cells are preferably mammalian cells, more preferably human cells. When the obtained exosomes are used in medicine for livestock, pets, etc., the host cells are preferably cells of the same species as the livestock, pets, etc. Examples of cell types include tissue stem cells (somatic stem cells) such as mesenchymal stem cells, neural stem cells, hematopoietic stem cells, bone marrow stem cells, skeletal muscle stem cells, and dental pulp stem cells; skin cells (fibroblasts, etc.), kidney cells, Tissue-derived cells such as hepatocytes, cardiomyocytes, pancreatic cells, osteocytes, chondrocytes, adipocytes; epithelial cells, endothelial cells, iPS cells, etc. Among these, mesenchymal stem cells (adipose tissue-derived mesenchymal Preferred are stem cells, umbilical cord-derived mesenchymal stem cells, bone marrow-derived mesenchymal stem cells, etc.), neural stem cells, fibroblasts, kidney cells (fetal kidney cells, etc.), and the like.

The present invention may further include a method in which the above-described method for producing exosomes containing a target nucleic acid is combined with a method for expressing a specific membrane protein or the like that will serve as a targeting moiety on the surface of the exosome. By using such a method, it becomes possible to specifically deliver a desired nucleic acid to a target tissue or cell and make it function, thereby improving the effectiveness as a nucleic acid drug.

The specific membrane protein that serves as the targeting portion is not particularly limited, and a membrane protein that specifically binds to the tissue or cell to which the desired nucleic acid is to be delivered can be appropriately selected. For example, it is a ligand/receptor that specifically binds to a receptor/ligand expressed on the surface of a target cell. The targeting moiety is selected to target a specific tissue type (e.g., muscle, brain, liver, kidney, heart, lungs, eye, etc.) or to target a diseased tissue such as a tumor. can do.

Methods for expressing a specific membrane protein that will serve as a targeting moiety on the surface of exosomes can be any method conventionally known to those skilled in the art, and are not particularly limited, but examples include the following methods.

For example, a polynucleotide construct (eg, a DNA plasmid) for expressing a particular membrane protein is introduced into a host cell. Any suitable method can be used to introduce the polynucleotide construct into the host cell. For example, techniques such as using vectors such as viruses, plasmids, and artificial chromosomes, lipofection, liposomes, and microinjection can be used. Among these, it is preferable to use vectors from the viewpoint of excellent introduction efficiency, and the vectors are not particularly limited as long as they can efficiently deliver membrane protein genes to host cells. Such vectors include, for example, lentivirus vectors, adeno-associated virus vectors, adenovirus vectors, herpesvirus vectors (e.g., herpes simplex virus vectors), poxvirus vectors, baculovirus vectors, papillomavirus vectors, papovavirus vectors. Examples include viral vectors such as vectors (eg, SV40); plasmid vectors; phagemid vectors; cosmid vectors; bacteriophages such as lambda phage and M13 phage. Among these, for the purpose of permanently expressing membrane protein genes, lentivirus vectors, adeno-associated virus vectors, and adenovirus vectors are preferred, lentivirus vectors and adeno-associated virus vectors are more preferred, and lentivirus vectors are preferred. More preferred. Furthermore, when the purpose is to transiently express a membrane protein gene, it is preferable to use a plasmid vector.

The polynucleotide construct includes a suitable promoter sequence so that the encoded membrane protein is expressed in the host cell. A signal peptide sequence is also included so that the protein is incorporated into the membrane of the endoplasmic reticulum when produced. Membrane proteins are then transported to the exosomal/lysosomal compartment before being incorporated into exosomes. The signal sequence is typically the signal peptide sequence of an exosome transmembrane protein.

A commercially available kit or the like may be used to express a specific protein or the like that will serve as a targeting moiety on the surface of the exosome. For example, XStamp (registered trademark) from SBI System Biosciences can be used. According to XStamp^{®}, by cloning a gene encoding a target protein into this vector and transfecting it into a host cell, it is possible to express various proteins on the surface of exosomes produced by that cell.. That is, by fusing a specific protein to the C1C2 domain of MFG-E8, which is a protein localized on the surface of exosomes, the above-mentioned specific protein can be displayed on the surface of exosomes.

### [Exosome production/secretion step]

In this step, the recombinant host cells obtained in the above step are cultured to produce and secrete exosomes containing the target nucleic acid. In addition, if the above step is combined with a method of expressing a specific membrane protein that will serve as a targeting moiety on the surface of the exosome, in this step, the recombinant host cells obtained in the above step are cultured, and the target Exosomes containing nucleic acids and expressing specific membrane proteins are produced and secreted.

In the present invention, the method for culturing recombinant host cells is not particularly limited as long as it is suitable for each cell, and methods conventionally known to those skilled in the art can be used. For example, when the recombinant host cell is a mammalian cell, the recombination is performed at a temperature of 30° C. to 37° C. in an environment of 2% to 7% CO2 and 5% to 21% O2. When a nucleic acid is transiently expressed in a recombinant host cell, the period during which the recombinant host cell releases the target exosome is about 1 to 4 days after introduction of the nucleic acid, so the culture period should be adjusted accordingly. and collect the culture supernatant. In addition, when the nucleic acid is expressed in a recombinant host cell, the cells are passaged, expanded, etc. depending on the state of the cells, and the culture supernatant is collected.

The medium used for cell culture is not particularly limited and can be selected from those conventionally known to those skilled in the art for each type of cell. From the viewpoint of using exosomes derived from the culture supernatant for humans, etc., a serum-free medium that does not contain animal serum is preferable. In addition, when the extracellular vesicles derived from the culture supernatant are used for purposes other than administration to humans, the culture medium may contain animal-derived serum.

The serum-free medium is not particularly limited as long as it does not contain animal serum. A known basic medium containing additives other than animal serum can be used. The composition of the basic medium can be appropriately selected depending on the type of cells to be cultured. For example, Minimum Essential Medium (MEM) such as Eagle's Medium, Dulbecco's Modified Eagle's Medium (DMEM), Minimum Essential Medium α (MEM-α), Mesenchymal Stem Cell Basal Medium (MSCBM), Ham's F-12 and F -10 medium, DMEM/F12 medium, Williams medium **E,** RPMI-1640 medium, MCDB medium, 199 medium, Fisher medium, Iscove's modified Dulbecco's medium (IMDM), McCoy's modified medium, and the like.

Other additives added to the basic medium include amino acids, inorganic salts, vitamins, and other additives such as carbon sources and antibiotics. The concentration of these additives used is not particularly limited, and they can be used at commonly used concentrations.

The method for producing exosomes containing nucleic acids, which is the object of the present invention, may further include an "exosome purification step" in addition to the above steps. In this step, the exosomes produced and secreted by the cells in the above exosome production/secretion step can be recovered from the culture medium by any appropriate method. Exosomes can be purified using methods conventionally known to those skilled in the art. The exosome purification method employed in this step is not limited, but includes, for example, the following method.

Preparations of exosomes can be prepared from the cell culture or supernatant obtained in the above steps by centrifugation, filtration, or a combination of these methods. For example, large particles can be pelleted and removed by low-speed centrifugation (less than 20,000 x g), followed by pelleting exosomes by high-speed centrifugation (100,000 x g or more), and filtered using a filter with a pore size of about 0.22 µm, for example. Purification can be performed by size filtration using a filtrate, density gradient centrifugation, or a combination of these methods.

### (Polynucleotide)

One embodiment of the present invention is a polynucleotide comprising a nucleic acid sequence of a target nucleic acid and a signal nucleic acid sequence represented by SEQ ID NO: 1. When a recombinant host cell is prepared by introducing the polynucleotide of the present invention into a host cell, the nucleic acid is selectively incorporated into exosomes within the host cell, and the exosome incorporating the nucleic acid is secreted into the culture medium.. The polynucleotide of the present invention is a construct in which a signal nucleic acid sequence (ELS) represented by SEQ ID NO: 1 is inserted into a non-coding region, and when introduced into a host cell, the nucleic acid is It is selectively incorporated into exosomes, and exosomes containing the nucleic acid are produced. The position where the ELS is inserted is not particularly limited as long as it is within the non-coding region, but it is preferably within the 3'UTR on the downstream side of the nucleic acid.

The polynucleotide of the present invention comprises a nucleic acid sequence of a target nucleic acid and a signal nucleic acid sequence represented by SEQ ID NO: 1, wherein the signal nucleic acid sequence represented by SEQ ID NO: 1 is located within a non-coding region. Preferably it is a nucleotide. Furthermore, the polynucleotide of the present invention includes a nucleic acid sequence of a target nucleic acid and a signal nucleic acid sequence represented by SEQ ID NO: 1, and the signal nucleic acid sequence represented by SEQ ID NO: 1 is located downstream of the nucleic acid. More preferably, it is a polynucleotide located within the 'UTR.

Regarding the signal nucleic acid sequence (ELS) represented by SEQ ID NO: 1, one or more (for example, 1 to 7, 1 to 6, 1 to 5, 1 to 4, 1 Even if a polynucleotide has a deletion, substitution, insertion, and/or addition of up to 3 nucleotides, 1 to 2 nucleotides, 1 nucleotide, etc., when it is introduced into a vector and transformed into a host cell, the host cell Any polynucleotide that can selectively incorporate a target nucleic acid into exosomes within cells is included within the scope of the present invention. Furthermore, combinations of two or more of these deletions, substitutions, insertions, and additions may be included at the same time. Generally, the smaller the number of nucleotide deletions, substitutions, insertions, and/or additions, the better. Preferred polynucleotides in the present invention also include polynucleotides capable of hybridizing to its complementary sequence of SEQ ID NO: 1 under stringent hybridization conditions, for example. Here, "stringent conditions" are as described above.

The polynucleotide of the present invention preferably further includes an operably linked promoter sequence, and includes known promoter sequences such as enhancer sequences, Kozak sequences, and appropriate polyadenylation signal sequences that assist in transcription of mRNA, translation into proteins, etc. May contain arrays.

### (Vector)

One embodiment of the present invention is a vector having a polynucleotide containing a nucleic acid sequence of a target nucleic acid and a signal nucleic acid sequence represented by SEQ ID NO: 1. Another embodiment of the present invention is an expression vector system that allows cloning in which a signal nucleic acid sequence represented by SEQ ID NO: 1 is inserted into a target nucleic acid.

That is, the vector of the present invention is a vector used to introduce a polynucleotide containing a nucleic acid sequence of a target nucleic acid and a signal nucleic acid sequence represented by SEQ ID NO: 1 into a host cell. The vector of the present invention comprises a nucleic acid sequence of a target nucleic acid and a signal nucleic acid sequence represented by SEQ ID NO: 1, and the signal nucleic acid sequence represented by SEQ ID NO: 1 is located within a non-coding region of a polynucleotide. It is preferable that the vector has the following. Furthermore, the vector of the present invention includes a nucleic acid sequence of a target nucleic acid and a signal nucleic acid sequence represented by SEQ ID NO: 1, and the signal nucleic acid sequence represented by SEQ ID NO: 1 is located downstream of the nucleic acid at the 3' More preferred are vectors with polynucleotides located within the UTR.

Examples of the vector of the present invention include vectors such as viruses, plasmids, and artificial chromosomes. More specifically, lentiviral vectors, adeno-associated viral vectors, adenoviral vectors, herpesvirus vectors (e.g., herpes simplex virus vectors), poxvirus vectors, baculovirus vectors, papillomavirus vectors, papovavirus vectors (e.g., Viral vectors such as SV40; plasmid vectors; phagemid vectors; cosmid vectors; bacteriophages such as lambda phage and M13 phage. Among these, when used for the purpose of permanently expressing a gene, lentivirus vectors, adeno-associated virus vectors, and adenovirus vectors are preferred, lentivirus vectors and adeno-associated virus vectors are more preferred, and lentivirus vectors are preferred. is even more preferable. Furthermore, when used for the purpose of transient gene expression, plasmid vectors are preferred.

The above vector can contain control sequences such as a promoter, enhancer, ribosome binding sequence, terminator, polyadenylation site, etc. so that the target nucleic acid can be expressed. In addition, if necessary, drug resistance genes (e.g. kanamycin resistance gene, ampicillin resistance gene, puromycin resistance gene, etc.), selection marker sequences such as thymidine kinase gene, diphtheria toxin gene, mCherry (red fluorescent protein), green fluorescent protein, etc. (GFP), β-glucuronidase (GUS), and a reporter gene sequence such as FLAG.

According to the present invention, once the desired nucleic acid to be loaded into exosomes has been determined, it can be introduced into a vector pre-equipped with a cloning site upstream of the non-coding region into which ELS has been inserted, resulting in a ready-to-use system. can. For example, if the target nucleic acid is a PCR product, a T vector or a cloning site combined with TOPO isomerase, and for other cases, a multi-cloning site is provided upstream of the non-coding region into which ELS has been inserted. Clone into a vector. By introducing this vector into host cells, exosomes containing the target nucleic acid can be produced and secreted within the host cells. Furthermore, by using a cassette (vector) into which a CMV promoter is inserted as a promoter, it is also possible to express RNA and protein from the desired nucleic acid in target cells. By using a cassette containing the U6 or H1 promoter, it is also possible to create short RNAs such as shRNA. An expression vector system that allows cloning in which a target nucleic acid is inserted in tandem is also an embodiment of the present invention.

### (Recombinant host cell)

One embodiment of the present invention is a recombinant host cell into which a polynucleotide containing a nucleic acid sequence of a target nucleic acid and a signal nucleic acid sequence represented by SEQ ID NO: 1 is introduced. This recombinant host cell is used to secrete exosomes containing the target nucleic acid.

In one embodiment of the present invention, a polynucleotide containing a nucleic acid sequence of a target nucleic acid, a signal nucleic acid sequence represented by SEQ ID NO: 1, and a polynucleotide encoding a specific membrane protein serving as a targeting moiety is introduced. and recombinant host cells. This recombinant host cell is used to secrete exosomes containing the desired nucleic acid and expressing a specific membrane protein on the surface that serves as a targeting moiety.

Regarding the specific content and preparation method of the recombinant host cell of the present invention, the explanation of the recombinant host cell preparation process in the above section of the method for producing exosomes containing the target nucleic acid can be applied as is.

### (Exosome)

One embodiment of the present invention is an exosome having a polynucleotide containing a nucleic acid sequence of a target nucleic acid and a signal nucleic acid sequence represented by SEQ ID NO: 1. The exosomes of the present invention can also be referred to as exosomes produced by the method for producing exosomes of the present invention described above.

One embodiment of the present invention comprises a polynucleotide comprising a nucleic acid sequence of a target nucleic acid and a signal nucleic acid sequence represented by SEQ ID NO: 1, and expresses a specific membrane protein serving as a targeting portion on the membrane surface. These are exosomes. The exosomes of the present invention can also be referred to as exosomes produced by the method for producing exosomes of the present invention described above.

By introducing a polynucleotide containing the nucleic acid sequence of the target nucleic acid and the signal nucleic acid sequence represented by SEQ ID NO: 1 into a host cell to prepare a recombinant host cell, and culturing this recombinant host cell, The exosome of the present invention containing the target nucleic acid can be produced and secreted from a recombinant host cell. In addition, a recombinant host cell preparation into which a polynucleotide containing the nucleic acid sequence of the target nucleic acid, a signal nucleic acid sequence represented by SEQ ID NO: 1, and a polynucleotide encoding a specific membrane protein serving as a targeting moiety is introduced. By culturing this recombinant host cell, the exosome of the present invention containing the target nucleic acid and expressing a specific membrane protein serving as a targeting portion on the membrane surface can be produced and secreted from the recombinant host cell. I can do it.

Regarding the specific content and manufacturing method of the exosome of the present invention, the explanation in the above section of the manufacturing method of an exosome containing a target nucleic acid can be applied as is.

The exosomes of the present invention can be suitably used for novel DDS technology to deliver desired nucleic acids to target cells. Exosomes are granules that are naturally secreted from various cells and covered with a lipid membrane with the same quality as cell membranes, and because they express membrane proteins that achieve immune tolerance, they are safe and stable in the body. Are better. Furthermore, in the case of drugs that act on cells in the brain, the administered DDS body needs to exceed the Brain Blood Barrier (BBB), but exosomes are covered with a lipid membrane that is the same as the cell membrane, so they cannot effectively cross the BBB. There are no restrictions on target organs that exist in existing DDS. Furthermore, the exosomes of the present disclosure are considered to have an advantage in that they can control their uptake into specific tissues and cells by expressing membrane proteins that interact with cells and are expressed in the membrane.

### (Pharmaceutical composition)

One embodiment of the present invention is a pharmaceutical composition containing the exosome of the present invention described above. Since the exosomes of the present invention can be loaded with desired nucleic acids, by using exosomes incorporating specific nucleic acids that are effective in treating various diseases, pharmaceutical compositions containing the exosomes can be used to treat various diseases. Suitably used for treatment. Furthermore, the pharmaceutical composition containing exosomes obtained by the present invention can be suitably used as a new DDS technique for delivering a desired nucleic acid to a target tissue or target cell. Regarding the specific content and manufacturing method of the exosome of the present invention, the explanation in the above section of the manufacturing method of an exosome containing a target nucleic acid can be applied as is.

Exosomes are granules covered with a lipid membrane that is the same as the cell membrane that is naturally secreted from various cells, and they also express membrane proteins that achieve immune tolerance, so they are safe and stable in the body. Excellent. Furthermore, in the case of drugs that act on cells in the brain, the administered DDS body needs to exceed the Brain Blood Barrier (BBB), but exosomes are covered with a lipid membrane that is the same as the cell membrane, so they cannot effectively cross the BBB. There are no restrictions on target organs that exist in existing DDS. Furthermore, by expressing membrane proteins that interact with cells, exosomes incorporating the desired nucleic acids of the present invention are considered to have the advantage of being able to control their uptake into specific tissues and cells. It will be done.

The pharmaceutical compositions of the invention may be administered by any suitable route of administration, including parenteral, intramuscular, intracerebral, intravascular (e.g. intravenous), intracardiac, intraventricular, intraperitoneal, subcutaneous, intranasal or transdermal administration. It can be formulated for use. Compositions for parenteral administration may include sterile aqueous solutions that may contain buffers, diluents and other suitable additives. Constructs of the invention may include in addition to the exosomes, such as pharmaceutically acceptable carriers, thickeners, diluents, buffers, preservatives, and other pharmaceutically acceptable carriers or excipients. It may be formulated into a pharmaceutical composition.

The pharmaceutically acceptable carrier is a pharmaceutically acceptable solvent, suspending agent, or any other pharmacologically inert vehicle for delivering exosomes to a subject. Typical pharmaceutically acceptable carriers include pregelatinized corn starch, polyvinylpyrrolidone, binders such as hydroxypropyl methylcellulose; lactose and other sugars, microcrystalline cellulose, pectin, gelatin, calcium sulfate, Fillers such as ethyl cellulose, polyacrylate, calcium hydrogen phosphate; magnesium stearate, talc, silica, colloidal silicon dioxide, stearic acid, metal stearate, hydrogenated vegetable oil, corn starch, polyethylene glycol, sodium benzoate, sodium acetate lubricants such as; disintegrants such as starch and sodium starch glycolate; wetting agents such as sodium lauryl sulfate.

The pharmaceutical composition of the present invention may further contain other additive components to the extent that the effects of the present invention are not impaired. For example, it may contain other pharmacologically active substances or may contain additional substances useful in physically preparing the various dosage forms of the pharmaceutical compositions of the invention, such as dyes, flavoring agents, preservatives, etc. It may also contain agents, antioxidants, opacifiers, thickeners, stabilizers, and the like.

The dosage of the pharmaceutical composition of the present invention is not particularly limited, and an appropriate dosage can be selected depending on various conditions such as the type of disease, age and symptoms of the patient, route of administration, purpose of treatment, and the presence or absence of concomitant drugs. It is possible to do so.

### (Treatment method)

One embodiment of the present invention is a method for preventing and/or treating a disease, which is characterized by administering the exosome of the present invention described above. Since the exosomes of the present invention can be loaded with desired nucleic acids, administration of exosomes incorporating specific nucleic acids that are effective in treating various diseases is effective for the prevention and/or treatment of various diseases. It is. In addition, regarding the specific content and manufacturing method of the exosome of the present invention, the explanation in the section of the above-mentioned method for manufacturing an exosome containing a target nucleic acid can be applied as is.

### EXAMPLES

Although the present disclosure will be specifically explained in the following examples, the present disclosure is not to be construed as limited by these examples.

### 1. Vector construction test for evaluating Exosome Localization Signal (ELS)

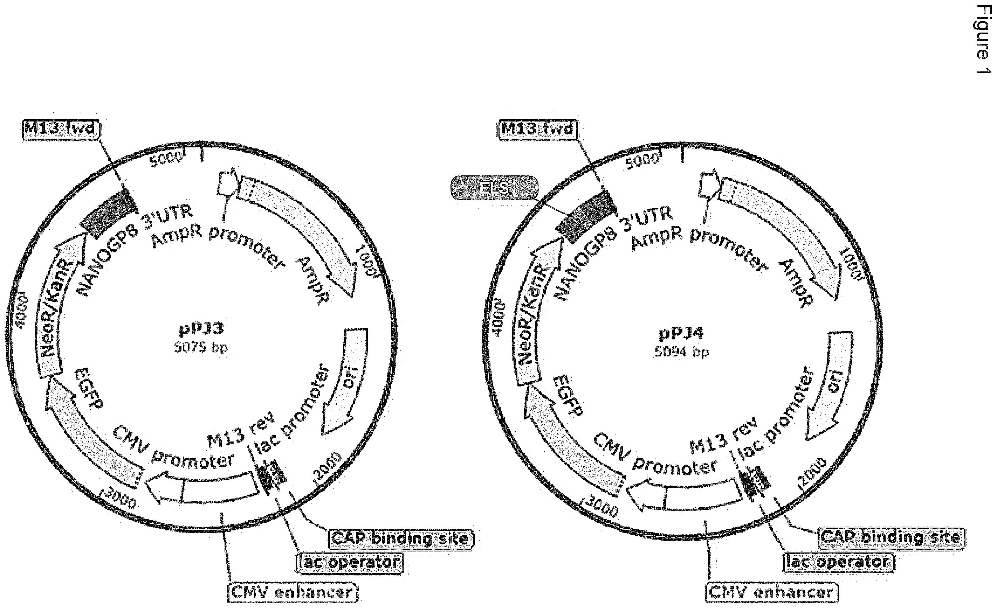

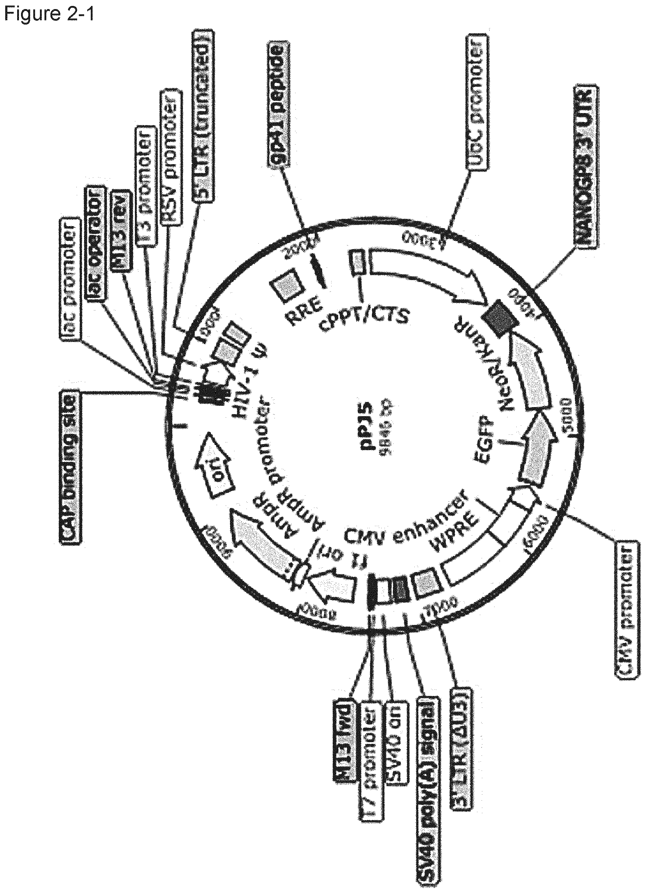

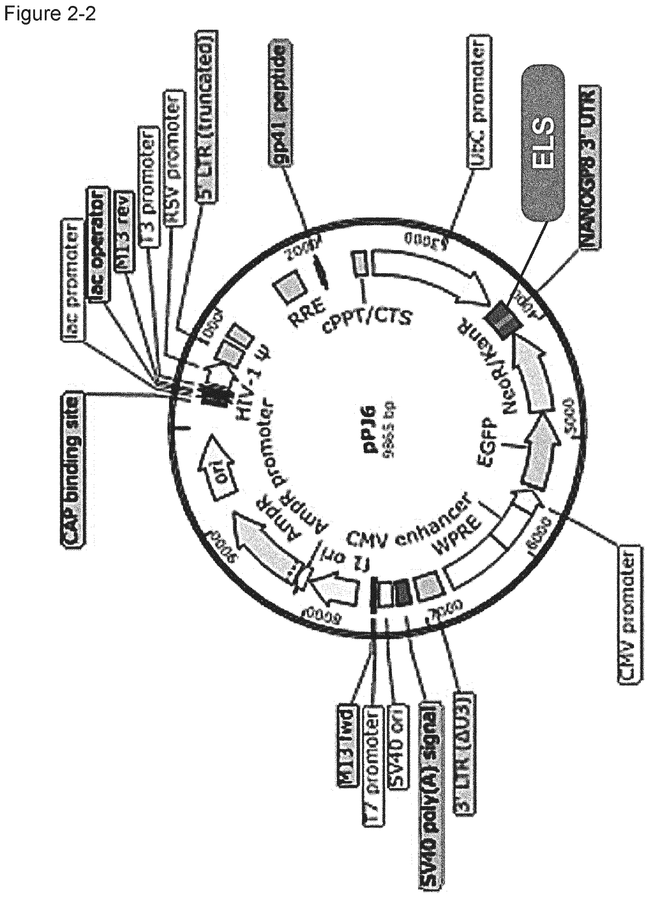

In this test, the vector was constructed in order to confirm the action of the Exosome Localization Signal (ELS) (SEQ ID NO: 1) composed of the DNA sequence consisting of 22 bp found by the present inventors. That is, a GFP expression cassette (pPJ4) with a 3'UTR containing ELS downstream of the GFP/Neo fusion protein gene initially expressed by the CMV promoter, and an equivalent GFP expression with a 3'UTR without ELS as a control. A cassette (pPJ3) was constructed (Figure 1). Furthermore, the above GFP expression cassette was inserted downstream of the UbC promoter on a lentiviral vector in the opposite direction to its transcription direction, and a construct containing ELS (pPJ5) and a construct containing no ELS (pPJ6) were constructed (Figure 2- 1, Figure 2-2). The specific content of the test is explained below.

The vectors used in this test are as follows.
- pRP[Exp]-EGFP/Neo-CMV){Tag/hNANOG[NM_024865.2]}: Vector containing CMV promoter and EGFP/Neo fusion gene
- Clone7A: 3'UTR of NANOGP8 that does not contain ELS Cloned vectors
- Clone7B: Vector in which the 3'UTR of NANOGP8 containing ELS was cloned
- pLenti Expression Construct: Lentiviral vector included in ViraPower Lentiviral Expression System (Invitrogen)
- pUC19: General empty vector

The E. coli strains used are as follows.
- DH5 (E. coli DH5 Competent Cells, 9057, manufactured by Takara Bio)

### (1) Construction of GFP expression cassette using pUC19

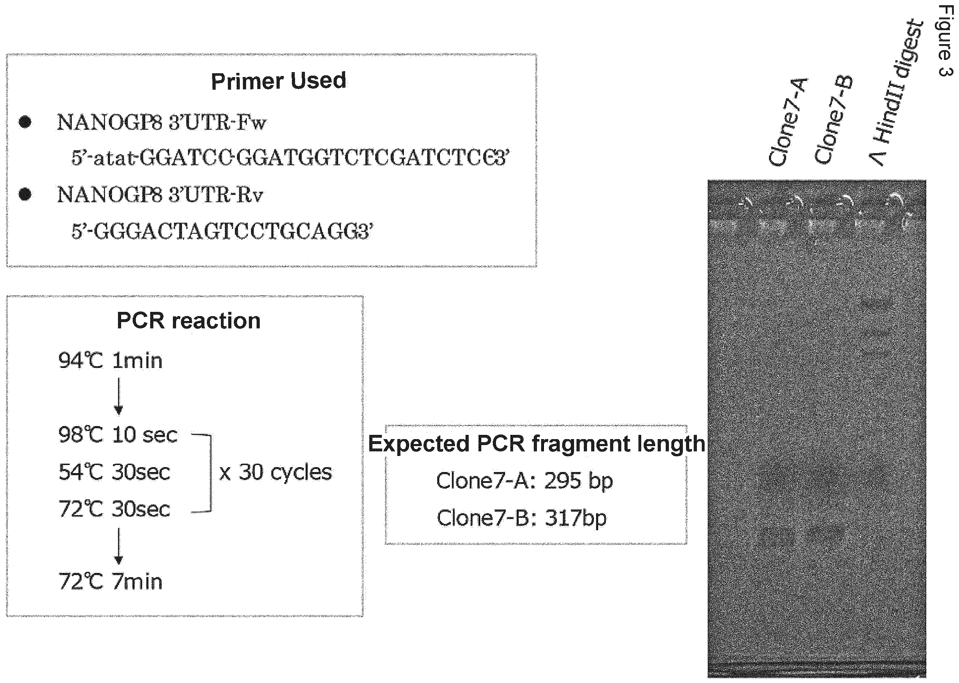

Clone 7A (vector in which the 3'UTR of NANOGP8 not containing ELS was cloned) and Clone 7B (vector in which the 3'UTR of NANOGP8 containing ELS was cloned) The above NANOG8 3'UTR region was confirmed. NANOGP8 3'UTR-Fw (SEQ ID NO: 2) and NANOGP8 3'UTR-Rv (SEQ ID NO: 3) were used as primers. As a result of PCR, a band was obtained at 295 bp for Clone 7A and 317 bp for Clone 7B (Fig. 3), which were both the expected PCR fragment lengths, confirming the existence of the expected fragment.

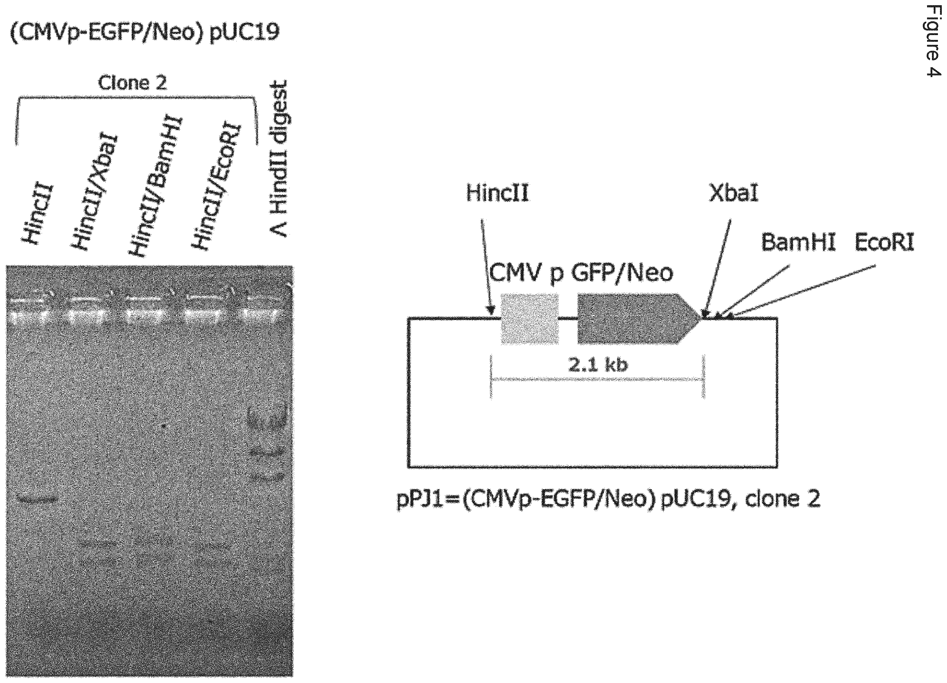

pRP[Exp]-EGFP/Neo-CMV){Tag/hNANOG[NM_024865.2]} was cut with Hincll and Xbal, and a band of approximately 2.1 kb containing the CMV promoter and EGFP/Neo fusion gene was cut out, and It was cloned into pUC19 cut with Xbal, and the ligation product was used to transform Escherichia coli. Clones 1 to 4 recovered therefrom were confirmed using restriction enzymes, and since clones 1, 2, and 4 were considered to be the desired constructs, clone 2 was further confirmed (FIG. 4). As a result, clone 2 was considered to be the desired construct, and thus clone 2 was used as pPJ1 in subsequent tests.

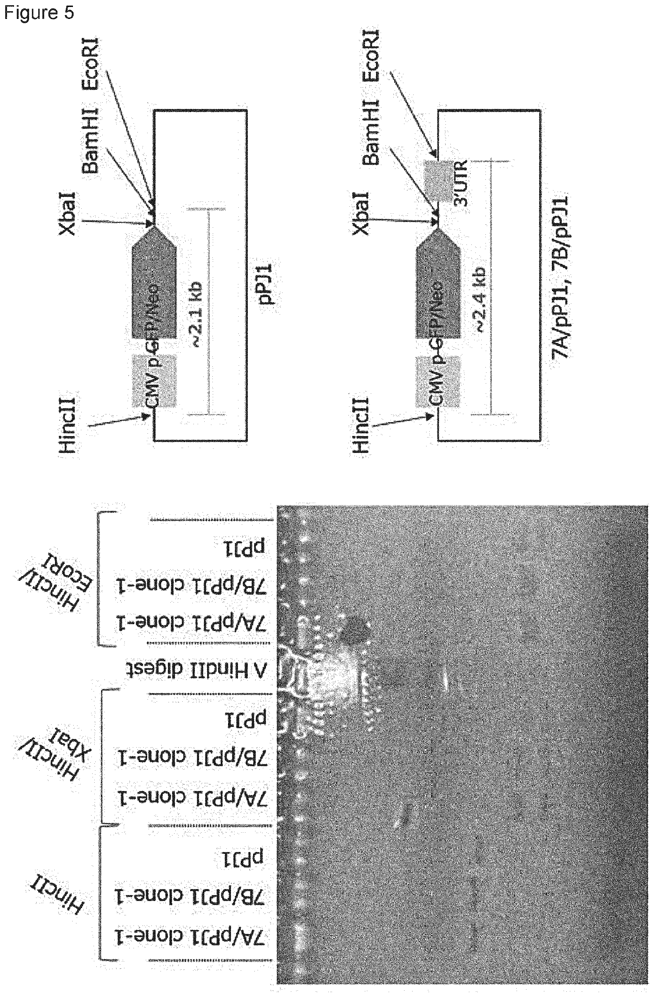

For Clone7A and Clone7B, PCR was performed using primers that can amplify the 3'UTR part of NANOGP8, and after cutting with EcoRI and BamHI, a DNA band of about 300 bp was cut out, and cloned into pPJ1, which was also cut with EcoRI and BamHI, and the ligation product was used to transform E. coli. When each clone 1 to 3 recovered therefrom was confirmed using restriction enzymes, clone 1 was considered to be the desired construct for both Clone7A and Clone7B (Fig. 5).

Clone 1 derived from Clone7A and clone 1 derived from Clone7B were sequenced using the M13-M4 primer to confirm whether the correct 3'UTR region was cloned downstream of the EGFP/Neo gene. As a result, it was confirmed that clone 1 derived from Clone7A and Clone7B had the expected 3'UTR sequence inserted. Therefore, a plasmid containing the Clone7A-derived 3'UTR (without ELS) was used as pPJ3, and a plasmid containing the Clone7B-derived 3'UTR (with ELS) was used as pPJ4 in subsequent tests.

### (2) Cloning of GFP expression cassette into lentiviral vector

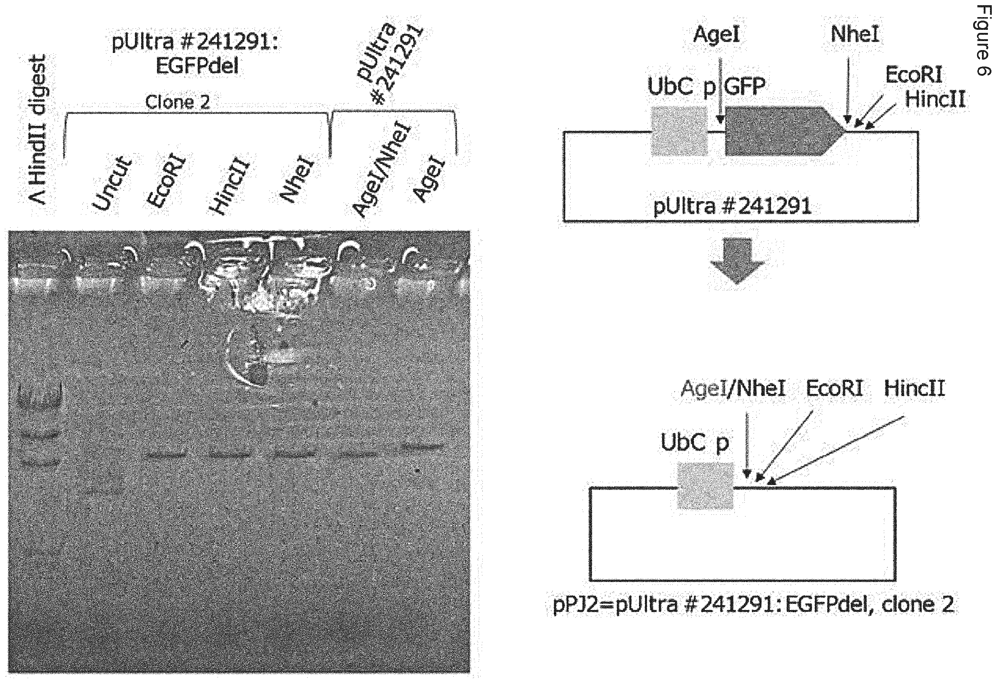

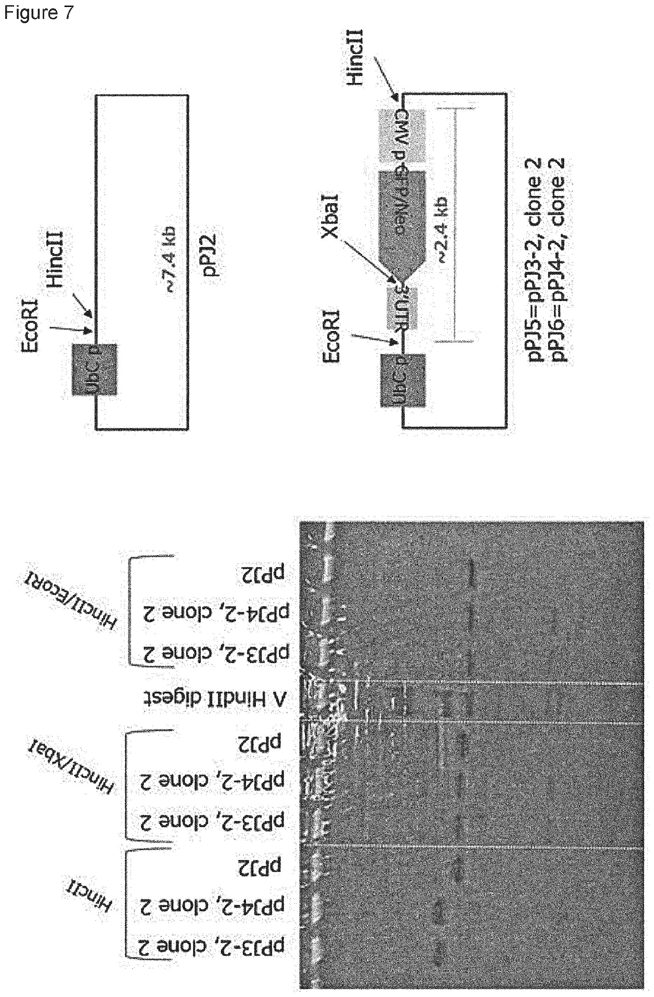

The lentiviral vector used this time already had EGFP downstream of the UbC promoter, so in order to remove this EGFP, it was cut with Agel and Nhel and then cut with Klenow fragment. After blunting, self-ligation was performed, and E. coli was transformed using this. The obtained clone was confirmed with restriction enzymes, and clone 2, which was the expected construct, was designated pPJ2 (Fig. 6). The GFP expression cassette portions contained in the above plasmids pPJ3 and pPJ4 were cut with Hincll and EcoRl, and cloned into pPJ2, which had been similarly cut with Hincll and EcoRI, to obtain respective clones. During cloning, the GFP expression cassette was inserted downstream of the UbC promoter in the opposite direction. A vector having a construct not containing ELS was designated pPJ5, and a vector having a construct containing ELS was designated pPJ6, as shown in FIG 7.

### 2. Exosome Localization Signal (ELS) evaluation

pPJ3, a plasmid containing the Clone7A-derived 3'UTR (without ELS) constructed above, or pPJ4, a plasmid containing the Clone7B-derived 3'UTR (including ELS), was used in human fetuses. Human Embryonic Kidney cells 293 (HEK293 cells) were transfected, and exosomes released into the culture supernatant were compared. In addition, pPJ5, a lentiviral vector containing the Clone7A-derived 3'UTR (without ELS) constructed above, or pPJ6, a lentiviral vector containing the Clone7B-derived 3'UTR (with ELS), was introduced into HEK293 cells. Lentiviral particles produced by transfection were transduced into human mesenchymal stem cells (human MSCs), and GFP DNA in exosomes was compared.

Specifically, pPJ3 or pPJ4 was transfected into HEK293 cells cultured in a 24-well plate using Lipofectamine (Lipofectamine Stem Transfection Reagent (Thermo Fisher, STEM0003)). pPJ1 was used as a control for creating a standard curve for qPCR. The amount of plasmid used in each transfection was 500 ng/sample, and the amount of lipofectamine was 2 µL/sample.

All culture supernatants were collected 30 and 48 hours after transfection or transduction. The collected culture supernatant was centrifuged at 200xg for 5 minutes to collect the supernatant, and further centrifuged at 10,000xg for 10 minutes to collect the supernatant. 3 µL of 7.5 M NaCl and 300 µL of 20% PEG were added to 150 µL of the supernatant, stirred, and then stored in a refrigerator overnight. Thereafter, the mixture was centrifuged at 10,000×g and 4° C. for 60 minutes, and the obtained exosome pellet was dissolved in 150 µL of sterile water (exosome isolation solution). After treating this exosome isolation solution at 100° C. for 10 minutes, DNA was collected using NucleoSpin Gel and PCR Cleanup (Takara Bio, U0609) and dissolved in 15 µL of sterile water.

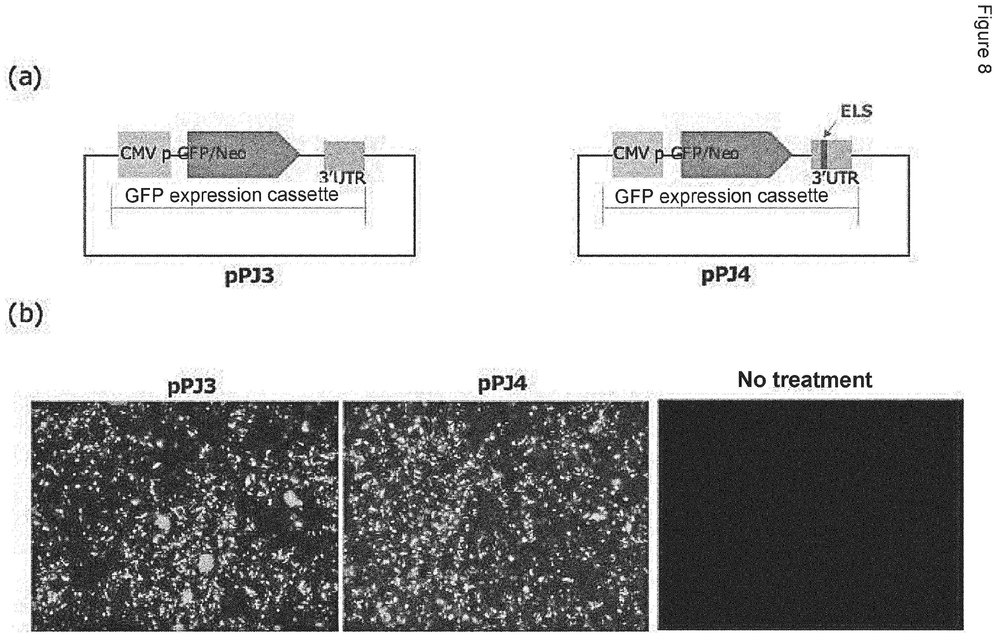

It was confirmed by qPCR how much of the introduced pPJ3- or pPJ4-derived construct was contained in the obtained exosomes. The target regions for PCR are outside the GFP expression cassette (Region 800) and inside the GFP expression cassette (Region 4000). For the region within (Region 4000), the primer set of SEQ ID NO: 6 and SEQ ID NO: 7 was used. The results are shown in Table 1 and FIG. 8 below.

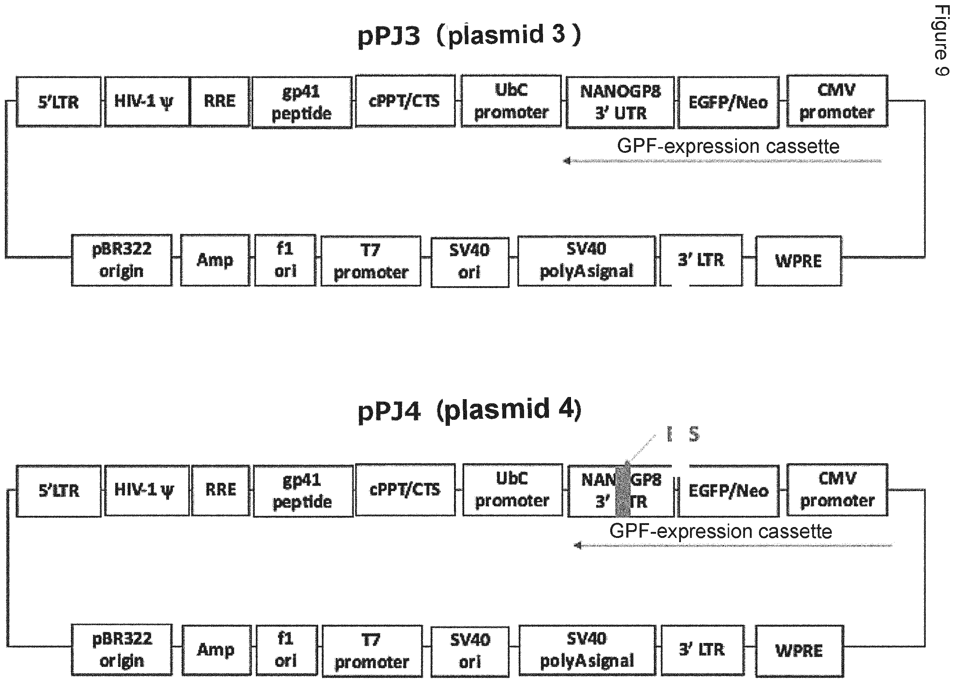

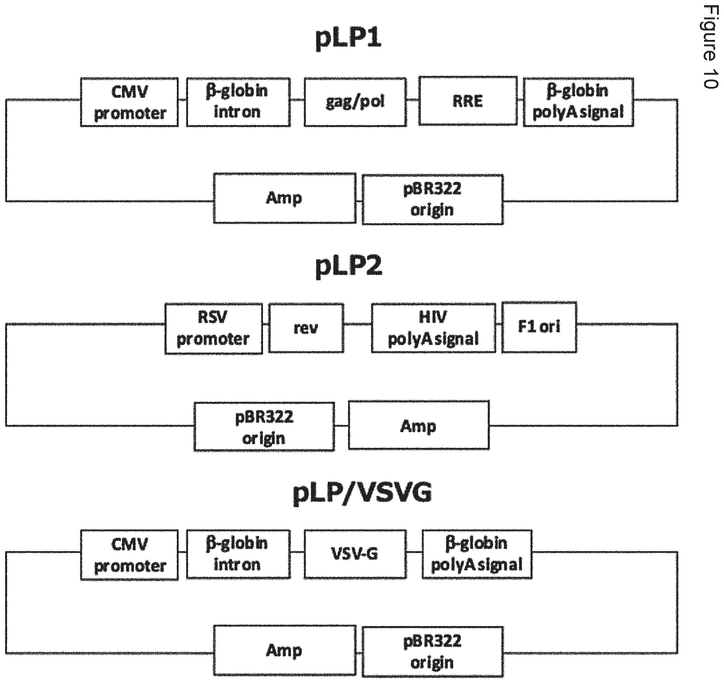

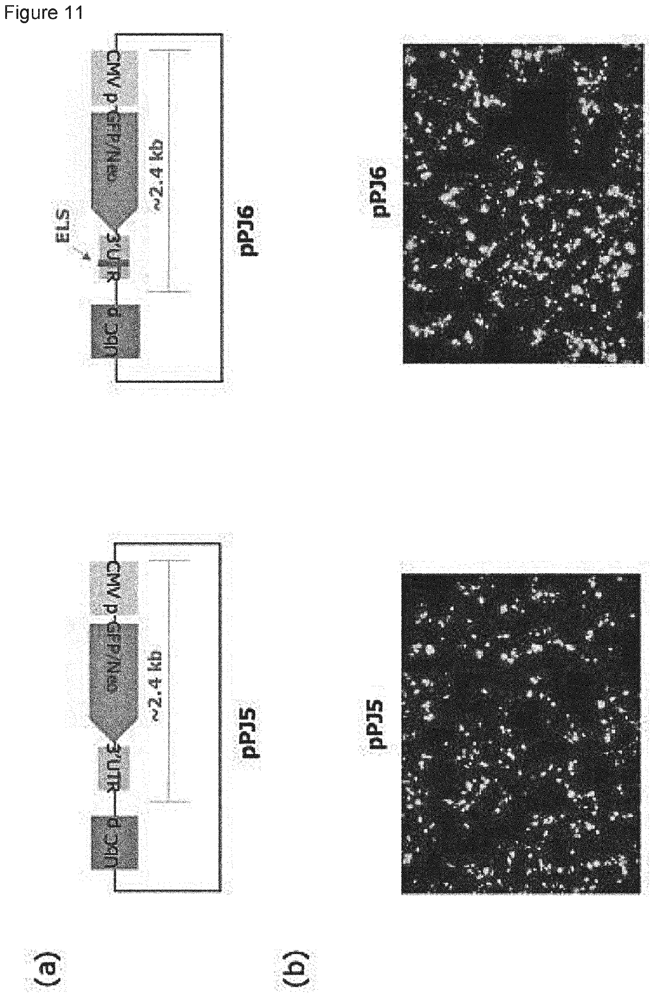

In addition to pPJ3 (plasmid 3) or pPJ4 (plasmid 4) (Figures 1 and 9), pLP1, pLP2, and pLP/VSVG (Figure 10) were introduced into 293FT cells, and lentiviral particles secreted over a certain period of time were transferred to polyethylene It was concentrated and recovered by glycol precipitation and centrifugation. Thereafter, the obtained lentiviral particles are processed into hMSCs to obtain hMSCs (rhMSCs) in which the lentiviral vector has been integrated into the genome. The rhMSCs are cultured for 48 hours, and exosomes are secreted into the culture medium. After recovery, the GFP expression cassette contained therein was quantified. The results are shown in Table 2 and FIG. 11.

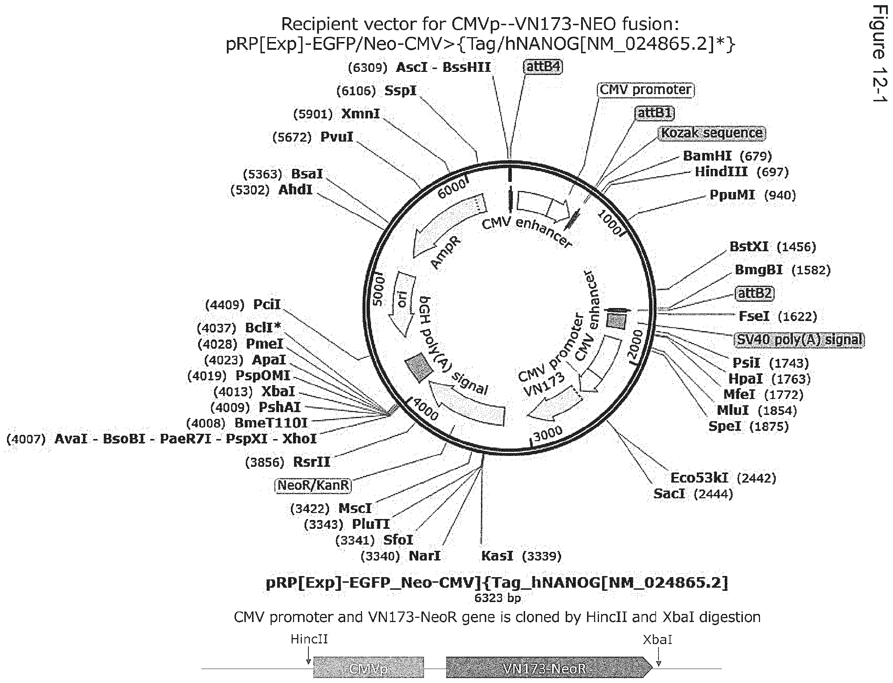

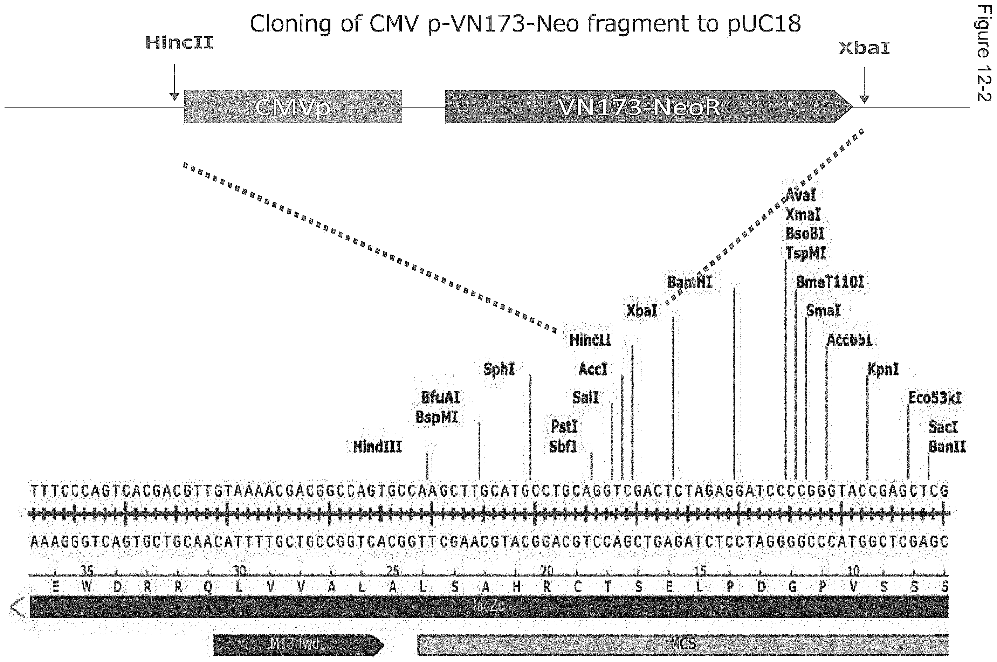

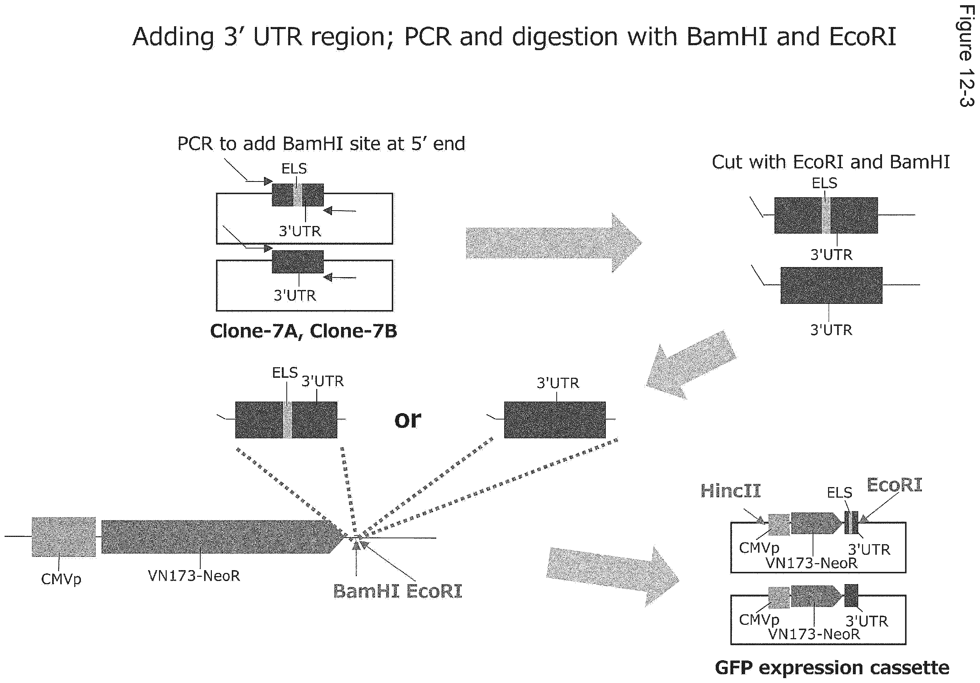

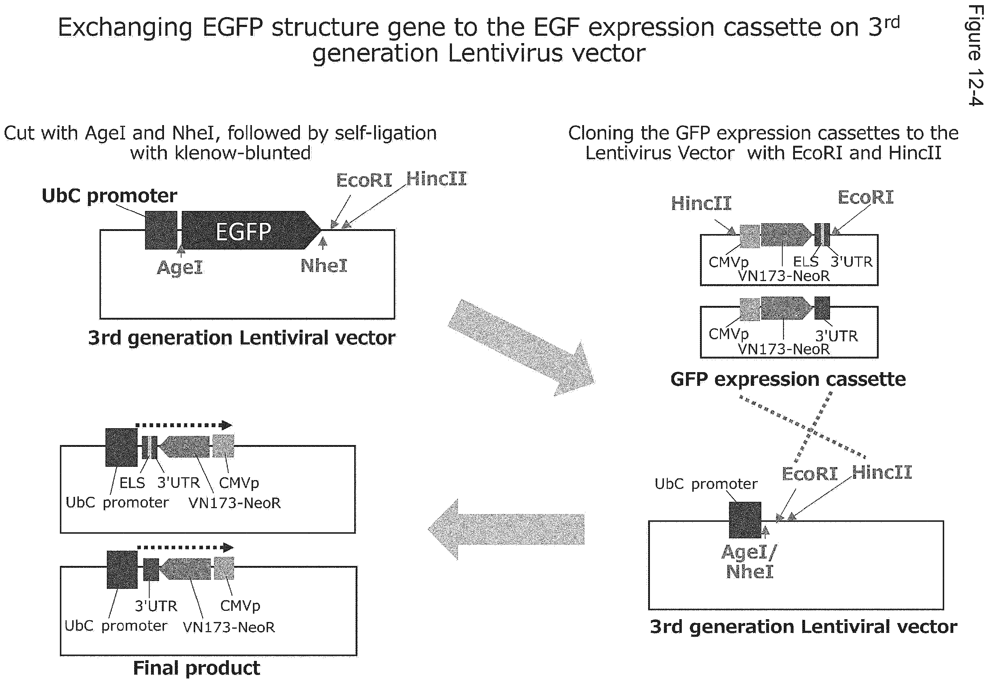

Figures 12-1 to 12-4 schematically show the flow of the vector construction described above.

**[Table 1]**

| Test date | Cells | Plasmid | Culture time after insertion | qPCR target | Amount present |
|---|---|---|---|---|---|
| 2024.10.13 | HEK293 | pPJ3 (without ELS) | 48 h | Region 800 | 30 pg |
| | | pPJ4 (with ELS) | | | 201 pg |
| | | pPJ3 (without ELS) | | Region 4000 | 50 pg |
| | | pPJ4 (with ELS) | | | 275 pg |
| 2022.02.21 | HEK293 | pPJ3 (without ELS) | 30 h | Region 800 | 311 pg |
| | | pPJ4 (with ELS) | | | 903 pg |
| | | pPJ3 (without ELS) | | Region 4000 | 284 pg |
| | | pPJ4 (with ELS) | | | 849 pg |

**[Table 2]**

| Cells | Vectors used to generate lentivirus | Culture time after lentivirus infection | qPCR target | Measurement sample | Amount present |
|---|---|---|---|---|---|
| Mesenchymal stem cells | pPJ5 (without ELS) | 48 h | Region 4000 | GFP mRNA in cells | 0.04 pg |
| | pPJ6 (with ELS) | | | | 0.06 pg |
| | pPJ5 (without ELS) | | | DNA in exosomes | 10.8 pg |
| | pPJ6 (with ELS) | | | | 13.8 pg |

As shown in Table 1 and Figure 8, cells transfected with pPJ4 containing ELS produced approximately 5 to 7 times more exosomes containing the transfected construct compared to cells transfected with pPJ3 without ELS. It was confirmed that a large amount was released. In other words, by introducing into cells a construct in which the ELS discovered by the present inventors is added to the tip of the desired DNA, it becomes possible to release exosomes incorporating the desired DNA into the culture supernatant. Ta.

As shown in Table 2 and FIG. 11, even when mesenchymal stem cells were used as host cells, the introduction of ELS promoted the transfer of the target nucleic acid to exosomes by about 30%.

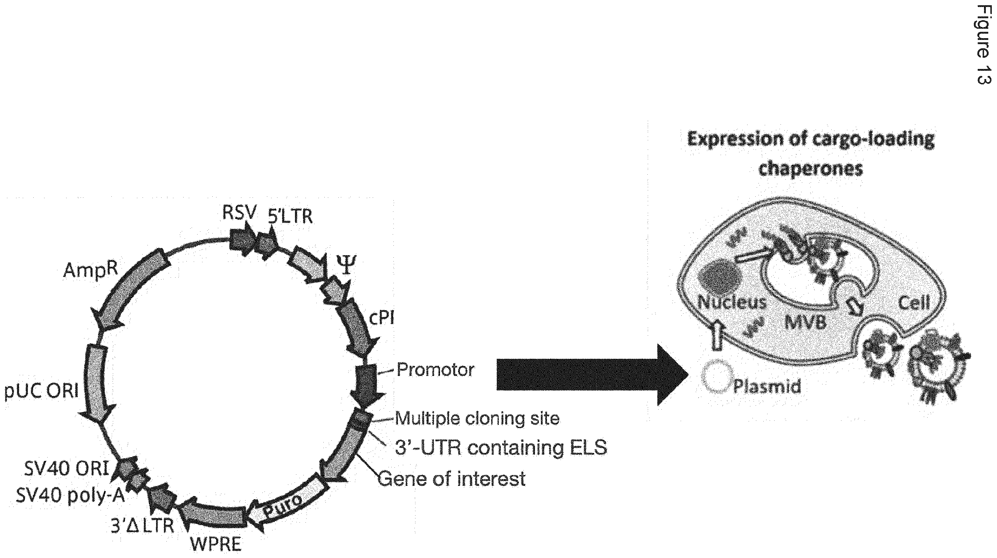

As described above, by the method of the present invention, a target nucleic acid is cloned into the upstream region of a non-coding region into which ELS has been inserted, and then transfected into a host cell, thereby transfecting exosomes produced by the cell. It is possible to introduce a target nucleic acid into a cell, but the mechanism is currently under study. A possible mechanism based on the results obtained so far is schematically shown in FIG. 13. That is, it is thought that RNA produced in host cells using the introduced vector as a template is reverse transcribed in the cytoplasm to become DNA, which is then transported to exosomes. This can be inferred from the fact that the gene sequence in exosomes is not necessarily a genome sequence, but an mRNA sequence after splicing.

### 3. Exosome Localization Signal (ELS) Evaluation-2

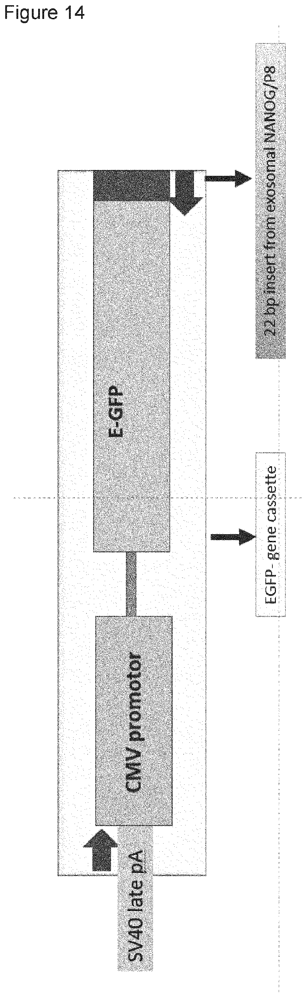

An expression vector containing the CMV promoter shown in Figure 14, the EGFP gene, and a 3'UTR containing the 22mer Exosome Localization Signal (ELS) (SEQ ID NO: 1) was used with Lipofectamine 2000 (Lipofectamine Transfection Reagent (HEK293 cells were transfected using Thermo Fisher). As a negative control, HEK293 cells were transfected using an expression vector containing the 3'UTR without the ELS instead of the 3'UTR containing the ELS. After transfection, exosomes released into the culture supernatant of HEK293 cells were compared.

Specifically, HEK293 cells cultured in a 24-well plate were transfected with the above expression vector using Lipofectamine 2000. The amount of plasmid used in each transfection was 500 ng/sample, and the amount of lipofectamine was 2 µL/sample.

All culture supernatants were collected 48 hours after transfection. The collected culture supernatant was centrifuged at 10,000×g for 30 minutes, and the supernatant was collected. 10 mL of the supernatant was transferred to a 50 mL tube, 20% PEG and 200 µL of 7.5 mM NaCl were added, and the tube was allowed to stand overnight at 4°C. The sample was centrifuged at 4° C. and 10,000×g for 60 minutes to obtain an exosome pellet. The pellet was suspended in PBS (H7.4, calcium and magnesium free) and further purified using CD63 antibody-conjugated magnetic beads (CD63 antibody-conjugated magnetic beads, Thermo Fisher Scientific, #10606D) to obtain exosomes (CD63 positive).. Note that CD63 is a protein belonging to the tetraspanin family member that is specifically expressed in exosome membranes, and is known as an exosome marker.

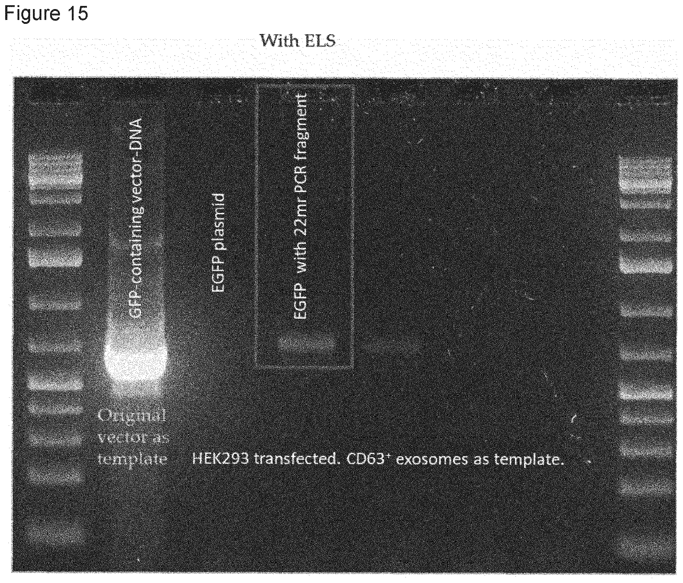

The extent to which the EGFP gene was contained in the obtained exosomes was confirmed by PCR. The primer set of SEQ ID NO: 8 and SEQ ID NO: 9 was used for PCR. The results are shown in FIG. 15.

As shown in Figure 15, HEK293 cells transfected with a gene cassette containing ELS were found to produce more exosomes containing GFP-DNA than HEK293 cells transfected with a gene cassette not containing ELS.

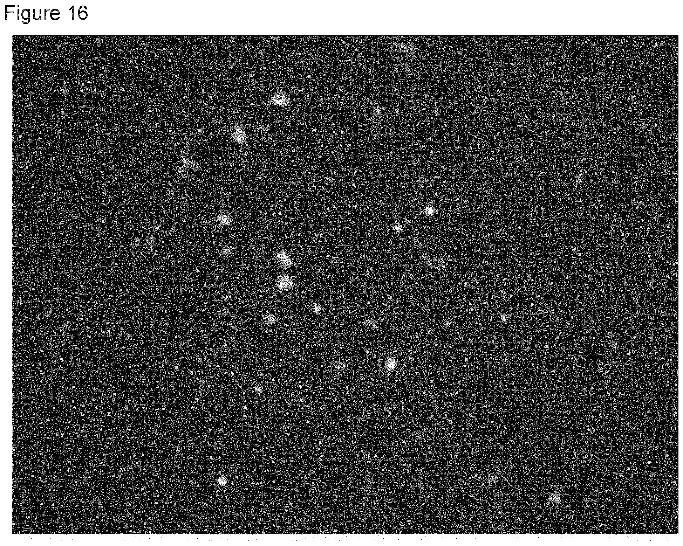

HEK293 cells were treated with the exosomes containing EGFP-DNA obtained above for 24 hours, and GFP expression in HEK293 cells was confirmed using a fluorescence microscope. As a control, HEK293 cells treated in the same manner as above with exosomes not containing EGFP-DNA were used. The observation results with a fluorescence microscope are shown in FIG. As shown in FIG. 16, in HEK293 cells treated with exosomes containing EGFP-DNA, it was confirmed that EGFP protein was expressed within the cells. By the method of the present invention, a target nucleic acid is cloned into the upstream region of a non-coding region into which ELS has been inserted, and transfected into a host cell, thereby injecting the target nucleic acid into exosomes produced by the cell. It has been shown that it is not only possible to introduce exosomes, but also to incorporate the exosomes obtained by this method into target cells, and also to express the target protein in the target cells.

Although test results using HEK293 cells are disclosed here, similar results have been obtained using cells other than HEK293 cells.

The specific sequences corresponding to SEQ ID NOS: 1 to 9 are as follows.
SEQ ID NO: 1: gctaaggaca acattgatag aa
SEQ ID NO: 2: atatggatcc ggatggtctc gatctcc
SEQ ID NO: 3: gggactagtc ctgcagg
SEQ ID NO: 4: accacgatgc ctgtagc
SEQ ID NO: 5: gcgcagaagt ggtcctg
SEQ ID NO: 6: gtgctcgacg ttgtcac
SEQ ID NO: 7: ccggatcaag cgtatgc
SEQ ID NO: 8: gtgggaggtttttaaagca
SEQ ID NO 9: acagacaatcggctgctctg

### INDUSTRIAL APPLICABILITY

According to the present disclosure, specific nucleic acids can be efficiently incorporated into exosomes in human cells (mesenchymal stem cells, human fetal kidney cells, etc.), and exosomes containing desired nucleic acids can be freely prepared. Exosomes obtained according to the present disclosure can be suitably used for novel DDS technology to deliver desired nucleic acids to target cells. Exosomes are granules that are naturally secreted from various cells and covered with a lipid membrane with the same quality as cell membranes, and because they express membrane proteins that achieve immune tolerance, they are safe and stable in the body. Are better. Furthermore, in the case of drugs that act on cells in the brain, the administered DDS body needs to exceed the Brain Blood Barrier (BBB), but exosomes are covered with a lipid membrane that is the same as the cell membrane, so they cannot effectively cross the BBB. There are no restrictions on target organs that exist in existing DDS. Furthermore, the exosomes of the present disclosure are considered to have an advantage in that they can control their uptake into specific tissues and cells by expressing membrane proteins that interact with cells.

## Claims

1. A method for producing exosomes containing a target nucleic acid, comprising
introducing a polynucleotide containing the nucleic acid sequence of the target nucleic acid and a signal nucleic acid sequence represented by SEQ ID NO: 1 into a host cell, to prepare a recombinant host cell and
culturing the recombinant host cell to produce and secrete exosomes containing the target nucleic acid.

2. A polynucleotide comprising a nucleic acid sequence of a target nucleic acid and a signal nucleic acid sequence represented by SEQ ID NO: 1.

3. A vector having a polynucleotide comprising a nucleic acid sequence of a target nucleic acid and a signal nucleic acid sequence represented by SEQ ID NO: 1.

4. An expression vector system capable of cloning a form in which a signal nucleic acid sequence represented by SEQ ID NO: 1 is inserted into a target nucleic acid.

5. A recombinant host cell into which a polynucleotide containing a nucleic acid sequence of a target nucleic acid and a signal nucleic acid sequence represented by SEQ ID NO: 1 is introduced.

6. The recombinant host cell according to claim 5, which is for producing exosomes containing the target nucleic acid.

7. An exosome having a polynucleotide comprising a nucleic acid sequence of a target nucleic acid and a signal nucleic acid sequence represented by SEQ ID NO: 1.

8. Exosomes produced by the production method according to claim 1.

9. A pharmaceutical composition comprising the exosome according to claim 7 or 8.
